# EUROPÄISCHE PATENTSCHRIFT

(11) **EP 1 239 813 B1**
(45) Veröffentlichungstag und Bekanntmachung des Hinweises auf die Patenterteilung: **13.04.2005**
(21) Anmeldenummer: 00989977.4
(22) Anmeldetag: 14.12.2000
(51) Int. Cl.: A61K 7/06, A61K 7/48, C12N 1/12

(54) **MAGNESIUM-ANGEREICHERTER EXTRAKT AUS DER BLAUALGE SPIRULINA UND SEINE VERWENDUNG IN KOSMETISCHEN MITTELN ZUR HAUT- UND KÖRPERPFLEGE**
EXTRACT OF BLUE-GREEN ALGA SPIRULINA AND THE USE THEREOF IN COSMETIC SKIN-CARE AND BODY CARE AGENTS
EXTRAIT DE L'ALGUE BLEUE SPIRULINA ET UTILISATION DANS DES AGENTS COSMETIQUES DESTINES AUX SOINS DE LA PEAU ET DU CORPS

(30) Priorität: 23.12.1999 DE 19962351
(43) Veröffentlichungstag der Anmeldung: 18.09.2002
(73) Patentinhaber: Henkel Kommanditgesellschaft auf Aktien, 40589 Düsseldorf (DE)
(72) Erfinder: SCHLOTMANN, Kordula, 40225 Düsseldorf (DE); WALDMANN-LAUE, Marianne, 40789 Monheim (DE); JASSOY, Claudia, 40235 Düsseldorf (DE); KAETEN, Melanie, 47608 Geldern (DE); KÖHLER, Eva-Maria, 14478 Potsdam (DE); PULZ, Otto, 14558 Bergholz-Rehbrücke (DE); KURTH, Elke, 14480 Potsdam (DE)
(86) Internationale Anmeldenummer: PCT/EP2000/012691
(87) Internationale Veröffentlichungsnummer: WO 2001/047473

(56) Entgegenhaltungen:
- EP-A- 0 049 632
- FR-A- 2 764 799
- GB-A- 2 274 465
- PATENT ABSTRACTS OF JAPAN vol. 011, no. 297 (C-448), 25. September 1987 (1987-09-25) & JP 62 087087 A (NIPPON SUPIRURINA KK), 21. April 1987 (1987-04-21)
- CHEMICAL ABSTRACTS, vol. 130, no. 4, 25. Januar 1999 (1999-01-25) Columbus, Ohio, US; abstract no. 35286, XP002164258 & GU, L. ET AL.: "Comparison of extracting methods for protein and several components from Spirulina platensis" ZHIWU SHENGLIXUE TONGXUN , Bd. 34, Nr. 3, 1998, Seiten 210-212,
- DATABASE WPI Section Ch, Week 198828 Derwent Publications Ltd., London, GB; Class C03, AN 1988-195274 XP002164260 & JP 63 133928 A (MATSUNAGA T), 6. Juni 1988 (1988-06-06)
- CHEMICAL ABSTRACTS, vol. 130, no. 24, 14. Juni 1999 (1999-06-14) Columbus, Ohio, US; abstract no. 329020, WANG, PEIJI ET AL.: "Skin- and hair-care preparation containing spirulina extracts" XP002164259 & CN 1 115 670 A (SHIPURUI CO. LTD.) 31. Januar 1996 (1996-01-31)

## Beschreibung

Die Erfindung betrifft einen Extrakt aus der Blaualge Spirulina und seine kosmetische Verwendung in Mitteln zur Haut- und Körperpflege.

Die kosmetische und dermatologische Behandlung der Haut erfolgt durch Cremes, Salben, Lotionen oder auch wässrige Liposomengele, die aufgrund ihrer Zusammensetzung an Fettstoffen und der damit verbundenen rückfettenden Eigenschaften einen positiven (glättenden) Effekt auf die Haut besitzen. Diese Mittel enthalten häufig auch wasserbindende Bestandteile, z. B. Harnstoff, die zum Wasserrückhalt in der Haut beitragen.

Die verhornte Epidermis bildet den Schutzschild der Haut. Damit diese Funktion optimal erfüllt wird, müssen die Hautzellen (Keratinozyten) den Prozess der sogenannten epidermalen Differenzierung durchlaufen. Nach der Teilung der Zellen in der Basalschicht wandern die Keratinozyten zur Hautoberfläche und machen dabei eine Reihe von Veränderungen durch, bis sie als tote, flache, kernlose Komeozyten die Homschicht (stratum comeum) bilden und schließlich abgeschuppt werden. Während der epidermalen Differenzierung werden verschiedenen Proteine mit spezifischen Funktionen ausgebildet. Hierzu zählen unter anderem Keratine, Involucrin, Filaggrin und Transglutaminase. Zur optimalen Ausbildung der Epidermis und der Homschicht ist es notwendig, dass diese Proteine koordiniert und in ausreichender Menge gebildet werden.

Das Adenosintriphosphat (ATP) ist ein zentrales Molekül des Energiehaushalts lebender Zellen, da es die Energiequelle vieler zellulärer Reaktionen darstellt. Der intrazelluläre ATP-Gehalt ist ein sensitiver Marker für die Vitalität und Aktivität von Zellen, da eine erhöhte zelluläre Aktivität, z. B. während der Proliferation (Zellvermehrung), mit einem erhöhten Bedarf und Verbrauch von ATP korreliert.

Bei bestimmten Hautkrankheiten wie Ichthyosis oder atopischer Dermatitis sowie bei Altershaut wurden Störungen im Differenzierungsprozess festgestellt und mit den entsprechenden klinischen Hautveränderungen in Verbindung gebracht. Hauterkrankungen können z. B. auf eine Verminderung des Filaggringehalts zurückzuführen sein. Auch in der Altershaut sind viele natürliche Komponenten vermindert, wie beispielsweise der Gehalt an Collagen, Elastin, bestimmten Ceramiden und freien Aminosäuren.

Die Altershaut wird kosmetisch in erster Linie mit Vitamin A-Derivaten oder Hydroxysäuren behandelt, die über eine Stimulation der Proliferation der Basalzellen in der Epidermis zu einer Verdickung der Epidermis und damit Glättung der Haut führen. Neuere Ansätze bestehen darin, die Proteine, die in der trockenen oder der Altershaut fehlen oder vermindert vorkommen, gezielt zu substituieren bzw. indirekt in die Stoffwechselprozesse einzugreifen, die bei trockener Haut oder mit zunehmendem Alter gestört sind, um diese zu normalisieren. Als Beispiel sei hier die Stimulation der Collagensynthese mit dem Zweck der Faltenreduzierung angeführt. Weiterhin werden beispielsweise Laminin, Substanzen zur Verlängerung der Lebenszeit von Hautzellen und bestimmte Extrakte zur Stimulierung der epidermalen Differenzierung eingesetzt. Hierbei handelt es sich teilweise jedoch um pharmakologisch wirksame Substanzen mit hohem Nebenwirkungspotenzial.

Es besteht also ein ständiger Bedarf an neuen, nachweislich bioaktiven Komponenten mit Wirkung auf die biochemischen Prozesse, die zu trockener Haut und zu Altershaut führen. Die Erfindung geht daher von der Aufgabenstellung aus, eine bioaktive Substanz zur Verfügung zu stellen, die die Synthese von Adenosintriphosphat (ATP) in den Hautzellen stimuliert. Die so bereitgestellte Energie steht der Haut für erhöhte Stoffwechselleistungen, z. B. die Hautemeuerung, oder Reparaturprozesse, z. B. bei UV-Schäden, zur Verfügung. Die Substanz soll außerdem gezielt die Synthese der Proteine, die in der trockenen oder in der Altershaut vermindert sind, stimulieren. Letztlich wird so das Hautbild bei trockener Haut oder Altershaut von innen her verbessert durch Regulation eines relevanten biochemischen Prozesses. Ebenso wird die Schutzfunktion der Haut wiederhergestellt.

Algenextrakte als kosmetischer oder dermatologischer Wirkstoff sind bereits seit langem bekannt. Ihre Verwendung in verschiedenen kosmetischen oder dermatologischen Präparaten ist beispielsweise in den Druckschriften JP 52 021 336 A, JP 54 037 835 A, JP 52 031 836 A, FR 2555444 A1 oder FR 2609246 A offenbart.

Die Patentschrift EP 0 652 763 B1 offenbart Blaualgenrohextrakte, die mindestens ein Porphyrinderivat als Wirkstoff enthalten.

Die Druckschrift FR 2764799 A1 beschreibt kosmetische Produkte, die einen Extrakt aus der marinen Mikroalge Phormidium enthalten und den intrazellulären ATP-Gehalt epidermaler Zellen in einer verschmutzten Atmosphäre bewahren oder sogar erhöhen.

Die Offenlegungsschrift CN 1 115 670 A offenbart wässrige Extrakte aus der Alge Spirulina, die Magnesium, weitere Mineralien, Aminosäuren und Zucker enthalten. Die Extrakte werden in kosmetischen Mitteln verwendet.

Die Offenlegungsschrift JP 62-087 087 A offenbart ein Verfahren zur Herstellung von Spirulina-Algen mit erhöhtem Gehalt an Magnesium, wobei die Algen in einem Nährmedium mit Magnesium kultiviert werden.

Algenextrakte enthalten kosmetisch oder sogar dermatologisch wirksame Komponenten wie Carotinoide, Proteine und Aminosäuren, Polyphenole, ungesättigte Fettsäuren und Vitamine, Polysaccharide, antioxidativ wirksame Mineralstoffe wie Selen und Zink, antioxidativ wirksame Enzyme wie Katalase, Superoxiddismutase und Peroxidase, Mineralstoffe wie Kalium, Magnesium und Eisen, mit pflegenden, stabilisierenden, feuchtigkeitsspendenden, entzündungslindemden, zellschützenden und zellstimulierenden Effekten. Die Zusammensetzung eines Algenextraktes variiert stark in Abhängigkeit von der Art der Alge, von den Wachstums- oder Kultivationsbedingungen und vom verwendeten Extraktionsverfahren. So enthalten die mittels schonender CO₂-Hochdruckextraktion gewonnenen lipophilen Spirulina-Extrakte überwiegend Fettsäuren und Carotinoide. In den wässrigen oder wässrig-glykolischen Spirulina-Extrakten liegen überwiegend uronsäurehaltige Polysaccharide, Polyphenole, Mineralstoffe und wasserlösliche Vitamine vor.

Es wurde nun überraschenderweise gefunden, dass bestimmte Extrakte aus einer mit Magnesiumsalzen angereicherten Blaualge vom Typ Spirulina die Anforderungen, die an einen kosmetischen Wirkstoff zur Stimulierung der Synthese von ATP und Differenzierungsproteinen in den Hautzellen gestellt werden, in hohem Maße erfüllen.

Intrazelluläres Magnesium ist größtenteils gebunden an ATP, Nukleinsäuren, Proteine und niedrigmolekulare Verbindungen, z. B. Citrate (T. Günther, Magnesium Bulletin 13, 46 - 52, 1991). Mg²⁺ wirkt als Modulator der Membranpermeabilität gegenüber Ca²⁺ und dessen transzellulärem Transport. Ca²⁺ ist das wichtigste Ion für die Keratinozytendifferenzierung. Außerdem induziert Ca²⁺ einen Anstieg der m-RNA zur Synthese von Involucrin, Loricrin, Transglutaminase, Keratinen und Filaggrin (D.D. Bikle, S. Pillai, Endocr. Rev. 14, 3 - 19, 1993). Collober et al. zeigten, dass sich eine Lösung, die Ca²⁺ und Mg²⁺ enthält, günstiger auf die Proliferation junger Fibroblasten und auf die Expression von Filaggrin auswirkt als eine Lösung, die nur Ca²⁺ enthält (I. Collober,

J. Wepierre, C. Montastier, M.S. Noel-Hudson, International Journal of Cosmetic Science 16, 149-160, 1994). Mg²⁺ optimiert oder potenziert sogar die Effekte von Ca²⁺ auf die Fibroblasten und Keratinozyten, was möglicherweise darauf zurückzuführen ist, dass es das lonengleichgewicht mit Ca²⁺ aufrecht erhält (J. Fine, P. Cole, J.S. Davidson, Biochem. J. 263, 371 - 376, 1989).

Ein erster Gegenstand der Erfindung ist die kosmetische Verwendung eines Blaualgenextraktes mit wenigstens 10 Gewichts-% Mg, bezogen auf die Trockenmasse des Extrakts, als Komponente zur Stimulierung der intrazellulären Synthese von ATP und Matrixproteinen, zur Stimulierung der Differenzierung von Keratinozyten in topischen Zubereitungen zur Reinigung und kosmetischen Pflege der Haut.

Der erfindungsgemäße Blaualgenextrakt wird bevorzugt aus dem Algentaxon Spirulina Arthrospira gewonnen. In einer bevorzugten Ausführung der Erfindung liegt die Trockenmasse des Extrakts bei 2 bis 10 Gewichts-% und der Magnesiumgehalt bei 0,3 bis 2 Gewichts-% des Extrakts.

Der Magnesiumgehalt des Algenextraktes wird in einer besonders bevorzugten Ausführungsform durch die Bedingungen der Algenkultivation, insbesondere die Zusammensetzung des Nährmediums, determiniert. Zur Aufrechterhaltung des durch die Kultivierung erzielten Magnesiumsgehaltes in der Biomasse kann es aber vorteilhaft sein, dem Extraktionsmittel Wasser vor der Extraktion Magnesiumsalze zuzusetzen.

Weiterhin offenbart ist ein Verfahren zur Herstellung eines Blaualgenextraktes, bei dem die Algen in einem wenigstens 0,01 Gewichts-% Mg-Ionen enthaltenden Nährmedium kultiviert, die gebildete Biomasse abgetrennt und getrocknet und dann mit Wasser oder einem Gemisch aus Wasser und mindestens einem wasserlöslichen, ein- oder mehrwertigen C₁₋₆-Alkohol extrahiert werden. Erfindungsgemäß besonders bevorzugte Extraktionsmittel sind Wasser und ein Gemisch aus Wasser und Glykol.

Weiterhin offenbart ist die Verwendung eines Blaualgenextraktes mit wenigstens 10 Gewichts-% Mg, bezogen auf die Trockenmasse des Extrakts, zur Herstellung von Mitteln zur dermatologischen Hautbehandlung.

Weiterhin offenbart ist ein Mittel zur dermatologischen Behandlung, zur Reinigung oder zur kosmetischen Pflege der Haut und der Haare, dadurch gekennzeichnet, dass ein Blaualgenextrakt mit wenigstens 10 Gewichts-% Mg, bezogen auf die Trockenmasse des Extrakts, in einer Menge von 0,01 bis 10 Gew.-% Trockensubstanz in einem Träger zur topischen Applikation auf der Haut oder auf dem Haar in Form einer wässrigen Tensidzubereitung, einer Emulsion, einer Salbe oder Creme, eines Gels, eines Puders, einer Maske, eines Matrixpflasters, eines Gelpflasters, eines Schaumes oder einer Aerosolzubereitung enthalten ist.

Weiterhin offenbart ist die Verwendung eines Blaualgenextraktes mit wenigstens 10 Gewichts-% Mg, bezogen auf die Trockenmasse des Extrakts, als Zusatz zur Stimulierung der intrazellulären Synthese von ATP und Proteinen in Zellkulturmedien.

Unter topischen Zubereitungen sind dabei solche Zubereitungen zu verstehen, die dazu geeignet sind, die genannten Wirkstoffe in feiner Verteilung und bevorzugt in einer durch die Haut resorbierbaren Form auf die Haut aufzubringen. Hierfür eignen sich z. B. wässrige und wässrig-alkoholische Lösungen, Sprays, Schäume, Schaumaerosole, Salben, wässrige Gele, Emulsionen vom O/W- oder W/O-Typ, Mikroemulsionen oder kosmetische Stiftpräparate. Besonders bevorzugt eignet sich als Träger ein wässriges Gel, eine Emulsion oder eine Mikroemulsion. Neben Wasser und physiologisch geeigneten Lösungsmitteln können u. a. pflegende Bestandteile, Öle, Wachse, Fette, rückfettende Substanzen, Verdickungsmittel, Emulgatoren, als Sonnenschutzfilter geeignete Substanzen und Duftstoffe enthalten sein. Im Sinne der Erfindung kann der Algenextrakt auch in Körperreinigungsmitteln wie z. B. Seifen, Duschbädem, Shampoos u. ä. verwendet werden. Für geeignete Formulierungsgrundlagen sei an dieser Stelle auf die in der Kosmetik üblichen Rezepturen verwiesen (K. H. Schrader, *Grundlagen und Rezepturen der Kosmetika,* 2. Aufl., Hüthig Buch Verlag, Heidelberg 1989, Seite 424-437, 442-451, 456-465).

Für die erfindungsgemäße Verwendung der Zusammensetzung zur topischen prophylaktischen oder kosmetischen Behandlung der trockenen Haut kann ein hoher Anteil an pflegenden Substanzen vorteilhaft sein. Gemäß einer bevorzugten Ausführungsform enthalten die Zusammensetzungen neben den in vielen Fällen ebenfalls Pflegewirkung aufweisenden tierischen und pflanzlichen Fetten und Ölen wie Olivenöl, Sonnenblumenöl, raffiniertes Sojaöl, Palmöl, Rapsöl, Sesamöl, Mandelöl, Boretschöl, Nachtkerzenöl, Jojobaöl, Kokosöl, Sheabutter (aus dem Samen der Pflanze Butyrospermium Parkii), Spermöl, Klauenöl, Rindertalg und Schweineschmalz noch weitere Pflegekomponenten. Dem Fachmann ist eine Vielzahl pflegender Wirkstoffe bekannt, die für diesen Zweck verwendet werden können. Hierzu zählen unter anderem:
- Fettalkohole mit 8 bis 22 C-Atomen:
   Die eingesetzten Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen bevorzugt von natürlichen Fettsäuren ab, wobei üblicherweise von einer Gewinnung aus den Estern der Fettsäuren durch Reduktion ausgegangen werden kann. Erfindungsgemäß einsetzbar sind ebenfalls Fettalkoholschnitte, die durch Reduktion natürlich vorkommender Fette und Öle, wie z. B. Rindertalg, Erdnußöl, Rüböl, Baumwollsaatöl, Sojaöl, Sonnenblumenöl, Palmkemöl, Leinöl, Rizinusöl, Maisöl, Rapsöl, Sesamöl, Kakaobutter und Kokosfett entstehen;
- Tierische und pflanzliche Proteinhydrolysate:
   Hierzu zählen insbesondere Elastin-, Kollagen-, Keratin-, Milcheiweiß-, Sojaprotein-, Seidenprotein-, Haferprotein-, Erbsenprotein-, Mandelprotein- und Weizenproteinhydrolysate, deren Kondensationsprodukte mit Fettsäuren sowie quaternisierte Proteinhydrolysate; die Verwendung pflanzlicher Proteinhydrolysate ist bevorzugt;
- Vitamine und Vitaminvorstufen wie Tocopherole, Vitamin A, Niacinsäure und Niacinsäureamid, weitere Vitamine des B-Komplexes, insbesondere Biotin, und Vitamin C. Weiterhin bevorzugt innerhalb dieser Gruppe von pflegenden Wirkstoffen sind Panthenol, dessen Derivate, insbesondere die Ester und Ether des Panthenols sowie kationisch derivatisierte Panthenole. Einzelne Vertreter sind beispielsweise Panthenoltriacetat, Panthenolmonoethylether und dessen Monoacetat sowie kationische Panthenolderivate;
- Mono-, Di- und Oligosaccharide wie beispielsweise Glucose, Galactose, Fructose, Mannose, Fruchtzucker und Lactose;
- Pflanzenextrakte, die üblicherweise durch Extraktion der gesamten Pflanze, in einzelnen Fällen aber auch ausschließlich aus Blüten und/oder Blättern der Pflanze, hergestellt werden. Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind. Erfindungsgemäß sind vor allem die Extrakte aus Eichenrinden, Brennessel, Hamamelis, Hopfen, Kamille, Klettenwurzel, Schachtelhalm, Weißdom, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt. Besonders bevorzugt sind die Extrakte aus Mandel, Aloe Vera, Kokosnuß, Mango, Aprikose, Limone, Weizen, Kiwi und Melone. Es können in den erfindungsgemäßen Mitteln auch Mischungen aus mehreren, insbesondere aus zwei, verschiedenen Pflanzenextrakten enthalten sein. Als Extraktionsmittel zur Herstellung der genannten Pflanzenextrakte können u. a. Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol, Propylenglykol und Butylenglykol und zwar sowohl als alleiniges Extraktionsmittel als auch in Mischung mit Wasser, bevorzugt. Die Pflanzenextrakte können erfindungsgemäß sowohl in reiner als auch in verdünnter Form eingesetzt werden;
- Honigextrakte, die in analoger Weise zu den Pflanzenextrakten gewonnen werden und üblicherweise 1 - 10 Gew.-%, insbesondere 3 - 5 Gew.-%, Aktivsubstanz enthalten;
- Ceramide; unter Ceramiden versteht man N-Acylsphingosin (Fettsäureamide des Sphingosins) oder synthetische Analoga solcher Lipide (sogenannte Pseudo-Ceramide), die das Wasserhaltevermögen des Stratum comeum deutlich verbessern;
- Phospholipide, beispielsweise Sojalecithin, Ei-Lecithin und Kephaline;
- Vaseline, Paraffin- und Silikonöle; zu letzteren zählen u. a. Dialkyl- und Alkylarylsiloxane wie Dimethylpolysiloxan und Methylphenylpolysiloxan, sowie deren alkoxylierte und quatemierte Analoga;
- Fettsäure- und Fettalkoholester, insbesondere die Monoester der Fettsäuren mit Alkoholen mit 3 bis 24 C-Atomen. Bei dieser Stoffgruppe handelt es sich um die Produkte der Veresterung von Fettsäuren mit 6 bis 24 C-Atomen wie Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Oxidation von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen;
- Silicone als pflegende Ölkomponenten, z. B. die Poly-(dimethylsiloxane) mit der INCI-Bezeichnung Dimethicone oder Cyclosiloxane mit der INCI-Bezeichnung Cyclomethicone.

Darüber hinaus können weitere kosmetische Wirkstoffe wie Sonnenschutzmittel, Hautfeuchthaltemittel, antimikrobielle Stoffe, deodorierende oder schweißhemmende Stoffe in den erfindungsgemäßen Hautpflegemitteln enthalten sein.

Schließlich können die erfindungsgemäße Zusammensetzungen alle bekannten und in solchen Zubereitungen üblichen Hilfs- und Zusatzstoffe enthalten, die zur Verbesserung der ästhetischen, anwendungstechnischen und kosmetischen Eigenschaften geeignet sind. Solche Stoffe sind z. B. natürliche oder synthetische Hydrocolloide wie wasserlösliche Cellulosederivate (Stärke), Pflanzengumme (Guar-Gum, Gummi Arabicum), Xanthan-Gum, Gelatine, Biopolymere oder auch synthetische wasserlösliche Polymere wie Polyvinylpyrrolidon oder Polyacrylsäure-Salze zur Verdickung der wässrigen Phase, Schichtsilikate (Bentonite), Veegum (Magnesium-aluminium-Silikat) oder verdickende feinteilige Kieselsäuren, Seifen, z. B. Calcium, Magnesium- oder Zinkseifen zur Verdickung der Ölphase, Farbstoffe, Pigmente, Duftstoffe, Konservierungsmittel, Komplexbildner, Puffersalze, Polyole wie Propylenglycol, Dipropylenglycol, Sorbit oder Glycerin und kosmetische Wirkstoffe wie Allantoin, Bisabolol und Extrakte aus tierischem Gewebe oder aus Mikroorganismen (z. B. Hefeextrakte).

Bevorzugt ist in der wässrigen Phase ein natürliches oder synthetisches Hydrocolloid in einer Menge von 0,01 bis 5 Gew.-% der Zusammensetzung enthalten.

Für die Herstellung kosmetischer oder pharmazeutischer Emulsionen zur Pflege und Behandlung der Haut werden hauptsächlich nichtionische Emulgatoren eingesetzt. Wegen ihrer positiven kosmetischen Eigenschaften sind besonders Phospholipide, Sterine, Alkyl(oligo)glycoside und die Fettsäureester von Zuckern, Zuckeralkoholen und Polyglycerin sowie deren Ethoxylate als Emulgatoren geeignet, wobei die genannten Verbindungen einzeln oder in Gemischen verwendet werden können.

Zu den als Emulgator geeigneten Phospholipiden zählen vor allem die Glycerophospholipide, die z. B. als Lecithine (Phosphatidylcholine) sowohl aus tierischen Produkten wie Eidotter als auch aus Pflanzensamen (z. B. Sojabohnen) gewonnen werden.

Unter Sterinen versteht man eine Gruppe von Steroiden, die am C-Atom 3 des Steroid-Gerüstes eine Hydroxylgruppe tragen und sowohl aus tierischem Gewebe (Zoosterine) als auch aus pflanzlichen Fetten (Phytosterine) isoliert werden. Beispiele für Zoosterine sind Cholesterin und Lanosterin. Beispiele geeigneter Phytosterine sind Ergosterin, Stigmasterin und Sitosterin. Auch aus Pilzen und Hefen werden Sterine, die sogenannten Mykosterine, isoliert.

Alkyl(oligo)glycoside sind Verbindungen der allgemeinen Formel RO(C₆H₁₀O)ₓH, in der R eine lineare gesättigte Alkylgruppe mit 8 bis 22 C-Atomen, C₆H₁₀O einen Glycosidrest, z. B. einen von Glucose abgeleitete Glucosidrest oder einen von Mannose oder Fructose abgeleiteten Mannosid- oder Fructosidrest und x dessen Oligomerisationsgrad darstellt. Bezüglich des Glycosidrestes gilt, daß sowohl Monoglucoside, bei denen ein Monosaccharid glycosidisch an einen Fettalkohol mit 8 bis 18 C-Atomen gebunden ist, als auch oligomere Glucoside mit einem Oligomerisationsgrad bis 10 geeignet sind. Der Oligomerisationsgrad ist dabei ein statistischer Mittelwert, der sich aus der quantitativen Zusammensetzung der Edukte ergibt. Bevorzugt eignen sich Alkyl-(oligo)-glycoside, in denen R eine Alkylgruppe mit 12 bis 18 C-Atomen ist und x einen Mittelwert von 1 bis 2 hat. Solche Alkylglycoside sind im Handel z. B. unter dem Warenzeichen Plantaren® oder Plantacare® erhältlich. Gemische aus Alkyl-(oligo) glucosiden und Fettalkoholen sind z. B. unter der Bezeichnung Montanov®68 oder Emulgade® PL 68/50 im Handel.

Fettsäureester von Polyglycerinen, Zuckern und Zuckeralkoholen wie Sorbitan und die Ethylenoxid-Anlagerungsprodukte dieser Verbindungen sind ebenfalls bekannte und im Handel erhältliche Emulgatoren. Darüber hinaus werden auch Fettalkohole und deren Ethylenoxid-Anlagerungsprodukte als Emulgatoren eingesetzt.

Weitere Emulgatoren, die zur Herstellung von Hautpflegeemulsionen im Sinne dieser Erfindung verwendet werden können, sind Silicon-Glykol-Polymere mit der INCI-Bezeichnung Dimethicone Copolyol, die z. B. von Dow Corning hergestellt werden.

Die Herstellung der erfindungsgemäßen kosmetischen Zusammensetzungen in Form von Emulsionen zur Pflege oder Behandlung der Haut erfolgt in üblicher Weise, indem man die lipidlöslichen Stoffe mit der Öl- oder Fettphase bis zur klaren Schmelze erwärmt und homogenisiert, die wasserlöslichen Bestandteile im Wasser löst und die erwärmte wässrige Phase unter Rühren in die Fettphase einemulgiert. Die Emulgierung kann unter Zuhilfenahme von Homogenisatoren erfolgen. Gegebenenfalls können Verdickungsmittel zugesetzt werden. Duftstoffe und temperaturempfindliche Wirkstoffe oder Pflanzenextrakte wie die erfindungsgemäßen Blaualgenextrakte werden bevorzugt erst nach dem Abbkühlen in die Emulsion eingearbeitet.

Der erfindungsgemäße Blaualgenextrakt kann auch in wässrigen Tensidzubereitungen zur Reinigung der Haut und des Haars eingesetzt werden. Als Tenside eignen sich in den erfindungsgemäßen Zubereitungen alle für die Verwendung am menschlichen Körper verträglichen oberflächenaktiven Stoffe. Hierzu kommen sowohl anionische als auch kationische, zwitterionische, ampholytische und nichtionische Tenside in Betracht.

Anionische Tenside sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium-, Magnesium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Amidethercarboxylate der Formel [R-NH(-CH₂-CH₂-O)ₙ-CH₂-COO]ₘZ, in der R für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel
   R-O(-CH₂-CH₂O)ₓ-SO₃H, in der R eine bevorzugt lineare Alkylgruppe mit 10 bis 18 C-Atomen und x = 0 oder 1 bis 12 ist,
- gemischte oberflächenaktive Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Kokosmonoglyceridsulfate.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen sowie Seifen.

Nichtionogene Tenside enthalten als hydrophile Gruppe z. B. eine Polyolgruppe, eine Polyalkylenglykolethergruppe oder eine Kombination aus Polyol- und Polyglykolethergruppen. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester sowie
- Anlagerungsprodukte von Ethylenoxid an Fettsäurealkanolamide.

Bevorzugte nichtionische Tenside sind Alkylpolyglykoside der allgemeinen Formel RO(C₆H₁₀O)ₓH, wie sie vorstehend bereits als nichtionische Emulgatoren beschrieben wurden. Der Alkylrest R enthält 8 bis 22 Kohlenstoffatome, wobei für den Einsatz als Tensid Alkylkettenlänge von 8 bis 16 C-Atomen bevorzugt sind. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Alkylsarcosin.

Beispiele für die in den erfindungsgemäßen Haut- und Haarreinigungsmitteln verwendbaren kationischen Tenside sind insbesondere quartäre Ammoniumverbindungen, Esterquats und Amidoamine.

Bevorzugte quatemäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quatemium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quatemierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Ein Beispiel für ein als kationisches Tensid einsetzbares quatemäres Zuckerderivat stellt das Handelsprodukt Glucquat® 100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die erfindungsgemäßen Blaualgenextrakte werden folgendermaßen gewonnen. Die Spirulina-Biomasse wird unter definierten Bedingungen in einem Kulturmedium mit einem Gehalt an Magnesiumionen von mindestens 0,01 Gewichts-% kultiviert. Mittels Filtration oder Separation und integrierten Waschvorgängen wird die Spirulina-Biomasse geerntet und unter schonenden Bedingungen sprühgetrocknet oder direkt der Extraktion zugeführt. Die geemtete oder auch sprühgetrocknete Spirulina-Biomasse wird mit heißem destillierten bzw. mineralstoffhaltigen Wasser oder einem Gemisch, bestehend aus Wasser und einem ein- oder mehrwertigen Alkohol extrahiert und dann mittels Filtration oder Separation abgetrennt. Der resultierende Extrakt wird einer Ultrafiltration unterworfen und mit einem Konservierungsmittel stabilisiert.

Der Einsatz des erfindungsgemäßen Blaualgenextraktes in kosmetischen Mitteln unterliegt erfindungsgemäß keinen Einschränkungen. Es kommen prinzipiell alle auf dem Markt befindlichen Arten von Mitteln, insbesondere die oben genannten Zubereitungen, in Betracht.

Die nachfolgenden Beispielrezepturen für die pflegende Behandlung der trockenen Haut und der Altershaut im Sinne der Erfindung sollen den Erfindungsgegenstand näher erläutern, ohne ihn auf diese Beispiele zu beschränken.

### Beispiele

Für die nachstehend beschriebenen Rezepturen und Untersuchungen wurden zwei verschiedene Extrakte aus dem Algentaxon Spirulina Arthrospira mit folgender Spezifikation verwendet:

| **Physikal. Eigenschaften** | **Methode/Gerät** | **Werte Algenextrakt SPHM 3001** | **Werte Algenextrakt SPHM 3002** | **Werte/Bereich Algenextrakt SPH 3000** |
|---|---|---|---|---|
| Farbe | visuell | gelblich-braun, transparent | gelblich-braun, transparent | gelblich-braun, transparent |
| Geruch | DAB 10 (V.3.1.6) | arteigen | arteigen | arteigen |
| Trockenmasse (105°C) | DAB 10 (V.6.22.N2) Absolutbestimmung | 4,2 % | 7,09 % | ca. 4 % |
| Spezifisches Gewicht (20°C) | DAB 10 (V.6.4) Pyknometer | 1,0174 g/cm³ | 1,0327 g/cm³ | 1,01 - 1,03 g/cm³ |
| pH-Wert | DAB 10 (V.6.3.1) Potentiometrische Bestimmung | 5,53 | 5,41 | 6,5 - 7,5 |
| Refraktionsindex (20°C) | DAB 10 (V.6.5) Refraktometer | 1,342 | 1,342 | 1,320 - 1,360 |
| Magnesium-Gehalt | Atomabsorptionsspektrometer | 0,6 Gew.-% | 1,2 Gew.-% | 0,12 Gew.-% |
| Magnesium-Gehalt, auf Trockenmasse bezogen | | 14 Gew.-% | 17 Gew.-% | 3 Gew.-% |
| | | erfindungsgemäß | erfindungsgemäß | nicht erfindungsgemäß, Vergleichssubstanz |

Alle Spirulina-Extrakte waren mit 0,75 Gew.-% Sepicide HB-2 konserviert. Der Spirulina-Extrakt SPH 3000 stammte aus Algen, die nicht unter Mg-anreichernden Bedingungen kultiviert worden waren und wurde hier zum Vergleich mit aufgeführt.

Im folgenden sind Beispielrezepturen für erfindungsgemäße Hautpflegemittel aufgeführt.

### Rezeptur 1:

| **Substanz** | **INCI-Bezeichnung** | **Hersteller** | **Menge [Gewichtsteile]** |
|---|---|---|---|
| Lipoid S-75-3 | Hydrogenated Lecithin | Lipoid GmbH | 1,5 |
| Stenol® 1618 | Cetearyl Alcohol | Henkel KGaA | 2,0 |
| Cetiol® SB-45 | Butyrospermium Parkii | Henkel KGaA | 1,0 |
| Cegesoft® C24 | Octyl Palmitate | Henkel KGaA | 6,0 |
| Prisorine® IPIS 2021 | Isopropyl lsostearate | Uniqema | 5,0 |
| Controx® KS | Tocopherol, Hydrogenated Palm Glycerides Citrate | Henkel KGaA | 0,05 |
| Konservierungsmittel | | | 0,4 |
| Polyacrylat | Carbomer | Goldschmidt | 0,5 |
| Methocel® E-4-M-premium | Hydroxypropylmethylcellulose | Dow Chemical | 0,1 |
| Algenextrakt SPHM 3002 | Algae extract | Institut für Getreideverarbeitung GmbH | 1,0 |
| 1,2-Propylenglykol | | | 5,0 |
| Glycerin | Glycerin | Henkel KGaA | 5,0 |
| Parfüm | Parfum | | 0,2 |
| Wasser, destilliert | Aqua | | ad 100 |

### Rezeptur 2:

| **Substanz** | **INCI-Bezeichnung** | **Hersteller** | **Menge [Gewichtsteile]** |
|---|---|---|---|
| Montanov® 68 | Cetearyl Alcohol, Cetearyl Glucoside | Seppic | 5,0 |
| Myritol® 318 | Caprylic/Capric Triglycerides | Henkel KGaA | 5,5 |
| Lanette® 22 | Behenyl Alcohol | Henkel KGaA | 3,0 |
| Cutina® MD-V | Glyceryl Stearate | Henkel KGaA | 1,25 |
| Cetiol® V | Decyl Oleate | Henkel KGaA | 2,0 |
| Baysilon® M 350 | Dimethicone | Bayer AG | 0,5 |
| Generol® 122-N-E-10D | PEG-10 Soy Sterol | Henkel KGaA | 0,5 |
| Controx® KS | Tocopherol, Hydrogenated Palm Glycerides Citrate | Henkel KGaA | 0,05 |
| Konservierungsmittel | | | 0,2 |
| Polyacrylat | Carbomer | Goldschmidt | 0,4 |
| Talkum | Talc | | 0,8 |
| Algenextrakt SPHM 3002 | Algae extract | Institut für Getreideverarbeitung GmbH | 1,0 |
| Glycerin | Glycerin | Henkel KGaA | 4,5 |
| Parfüm | Parfum | | 0,2 |
| Wasser, destilliert | Aqua | | ad 100 |

### Rezeptur 3:

| **Substanz** | **INCI-Bezeichnung** | **Hersteller** | **Menge [Gewichtsteile]** |
|---|---|---|---|
| Safloröl AEH 7 | Carthamus Tinctorius | Lesieur GmbH | 3,0 |
| Myritol® PC | Propylenglycol Dicaprylate/Dicaprate | Henkel KGaA | 3,5 |
| Lanette® 22 | Behenyl Alcohol | Henkel KGaA | 2,5 |
| Cutina® GMS | Glyceryl Stearate | Henkel KGaA | 4,0 |
| Stenol® 1618 | Cetearyl Alcohol | Henkel KGaA | 2,0 |
| Isopropylstearat | Isopropylstearate | Henkel KGaA | 6,0 |
| Baysilon® M 350 | Dimethicone | Bayer AG | 1,0 |
| Controx® KS | Tocopherol, Hydrogenated Palm Glycerides Citrate | Henkel KGaA | 0,05 |
| Konservierungsmittel | | | 0,2 |

| Phase 2: Verdickerphase | | | |
|---|---|---|---|
| DRY-FLO-PLUS® | Aluminium Starch Octenyl Succinate | National Starch | 3,0 |
| Talkum | Talc | | 0,5 |
| 1,6-Hexandiol | Hexamethylene Glycole | BASF | 6,0 |
| Polyacrylat | Carbomer | Goldschmidt | 0,3 |
| Algenextrakt SPHM 3002 | Algae extract | Institut für Getreideverarbeitung GmbH | 1,0 |
| Parfüm | Parfum | | 0,3 |
| Wasser, destilliert | Aqua | | ad 100 |

### Nachweis der Stimulierung der Filaggrin-Synthese in Hautzellen der oberen Epidermisschichten durch einen erfindungsgemäßen Blaualgenextrakt

Der erfindungsgemäße Blaualgenextrakt wurde *in vitro* an Humanhautäquivalenten auf seine stimulierende Wirkung auf die Filaggrin-Synthese in Hautzellen der oberen Epidermisschichten getestet.

### 1. Herstellung des Humanhautäquivalents

Zur Herstellung des Humanhautäquivalents wurden aus neonataler Vorhaut isolierte Fibroblasten auf eine Matrix, bestehend aus einer Mischung aus chitosan-quervemetztem Collagen und Glucosaminoglucan, gesät und 14 Tage lang submers in Inserts (Transwell, COSTAR) kultiviert. Danach erfolgte die Einsaat von ebenfalls aus Vorhaut gewonnenen Keratinozyten auf das Dermis-Modell. Nach weiteren 7 Tagen submerser Kultivierung wurde die Menge des Kulturmediums verringert, so dass die Kulturen nur noch von der Unterseite mit Medium versorgt wurden (Air-Liquid-Interface). Auf diese Weise wurde die Ausbildung des Stratum comeums als Barriere an der Oberseite der Kultur gewährleistet. Die Kultivierung im Air-Liquid-Interface erfolgte für 14 Tage mit serumfreiem Medium.

### 1.2 Herstellung der Test-Cremes

Für die Hauttests wurde der erfindungsgemäßer Blaualgenextrakt Spirulina SPHM 3002 mit einem Magnesiumgehalt von 1,2 Gewichts-%, bezogen auf das Gewicht des Extrakts, verwendet.

Zur Durchführung der Hauttests wurde eine Testcreme gemäß der Beispielrezeptur Nr. 2 hergestellt, die 2 Gewichts-% Algenextrakt, bezogen auf die gesamte Creme, enthielt. Die Testcreme ohne Algenextrakt wurde als Placebocreme mitvermessen.

### 1.3 Durchführung der Hautkultur-Tests

Bei allen Versuchsreihen wurden Placebokontrollen mitgeführt. Pro Versuchsreihe (Placebocreme und Creme mit 2 Gewichts-% Algenextrakt) wurden jeweils drei Kulturen parallel eingesetzt. Jeder Test erstreckte sich über die Dauer von 10 Tagen. Nach der Anzuchtphase des Hautäquivalents wurde die Testcreme insgesamt je viermal appliziert: direkt nach der Fertigstellung sowie nach 3, 5 und 7 Tagen. Dazu wurden jeweils 5 µl Creme (entsprechend ca. 3,8 mg/cm²) appliziert und mit einem weichen Pinsel vorsichtig verteilt. Am 10. Tag nach der Anzuchtphase wurde aus der Mitte jeder Kultur eine kreisförmige Probe mit 8 mm Durchmesser ausgestanzt und als Kryoschnitt präpariert. Die Präparate wurden immunhistochemisch auf die Anwesenheit von Filaggrin untersucht. Der Filaggrin-Nachweis erfolgte mittels indirekter Immunfluoreszenz unter Verwendung von Fluorescein-Isothiocyanat (FITC). Dazu wurden die Kryoschnitte mit einem spezifischen Antikörper gegen Filaggrin behandelt und dieser mittels eines FITC-konjugierten Sekundärantikörpers markiert. Von den markierten Präparaten wurden Mikroskopaufnahmen angefertigt und qualitativ ausgewertet.

Die qualitative Auswertung der Mikroskopaufnahmen der Fluoreszenzanalyse zeigte, dass die Behandlung mit der Algenextrakt-haltigen Creme im Vergleich zur Placebocreme zu einem deutlichen Anstieg des Filaggringehalts in den tieferen Epidermisschichten der Hautäquivalente führte.

### Nachweis der Stimulierung der ATP-Synthese in normalen humanen epidermalen Keratinozyten (NHEK) durch einen erfindungsgemäßen Blaualgenextrakt

Der erfindungsgemäße Blaualgenextrakt wurde *in vitro* an normalen humanen epidermalen Keratinozyten (NHEK) auf seine stimulierende Wirkung auf die intrazelluläre ATP-Synthese getestet.

### 1. ATP-Nachweismethode

Der intrazelluläre ATP-Gehalt wird über bioluminometrische Messungen mit einem Luciferase-Assay erfasst. Dazu werden die Zellkulturen mittels eines Extraktionspuffers aufgeschlossen und das Zell-Lysat mit einem kommerziell erhältlichen Luciferase-Reagenz versetzt. Das dabei gebildete Biolumineszenzlicht wird mit Hilfe eines Lumineszenzmessgerätes erfasst und ist direkt proportional zur Menge des im Lysat vorhandenen ATP.

### 2. Versuchsdurchführung

### 2.1 Vorbereitung der Testlösungen

Die Tests wurden mit verdünnten Lösungen von verschiedenen hydrophilen Extrakten der Blaualge Spirulina platensis durchgeführt, und zwar mit Spirulina SPH 3000 (nicht erfindungsgemäß, Vergleichssubstanz) und den erfindungsgemäßen Extrakten Spirulina SPHM 3001 und Spirulina SPHM 3002, deren Spezifikationen vorstehend aufgeführt wurden.

Zur Herstellung der Testlösungen wurden die Algenextrakte mit der zur Kultivierung der Keratinozyten verwendeten Nährmediumslösung (KGM Nährmedium der Firma Cell Systems) verdünnt und homogenisiert. Die Testlösungen enthielten die Algenextrakte in Konzentrationen von 0,1 Gew.-%, 0,5 Gew.-% und 1,0 Gew.-%.

### 2.2 Vorbereitung der normalen humanen epidermalen Keratinozyten und Inkubation mit den Algenextrakt-Lösungen

Die Versuche wurden an normalen humanen epidermalen Keratinozyten (NHEK P 135 der Firma Cell Systems) durchgeführt. Es handelt sich um primäre Keratinozyten. Nach der Anzucht der Zellen im KGM Nährmedium wurden die Zellen in 96-Kavitätsmikrotiterplatten mit einer Dichte von ca. 2000 Zellen pro Kavität eingesät. Nach drei Tagen wurde das Anzuchtmedium gegen die verschiedenen Algenextrakthaltigen Testlösungen ausgetauscht.

In jeder Versuchsreihe wurden unbehandelte, das heißt Algenextrakt-frei inkubierte Zellkulturen mitgeführt und analysiert. Die Inkubation der primären Keratinozyten mit den einzelnen Testlösungen erfolgte anschließend für 3 und 6 Stunden im CO₂-Inkubator bei einer Temperatur von 37°C und 5 Vol.-% CO₂. Nach Ablauf der jeweiligen Inkubationszeiten wurde der ATP-Gehalt der Zellen analysiert (siehe unten). Nach 24 Stunden Inkubationszeit wurde die Vitalität (Zellproliferation/Metabolismus) der Kulturen mit Hilfe des MTT-Tests bestimmt (siehe unten).

### 2.3 Durchführung der ATP-Messungen

Nach den jeweils vorgesehenen Inkubationszeiten wurden die Kontroll- bzw. Testlösungen von den Keratinozyten durch vorsichtiges Waschen entfernt. Anschließend wurden 60 µl eines Lyse-Puffers in jede Kavität (well) pipettiert. Die Mikrotiterplatten wurden dann 5 Minuten bei Raumtemperatur geschüttelt. Der Lyse-Puffer bestand aus einer wässrigen Lösung von 10 mM TRIS (2-Amino-2-hydroxymethyl-1,3-propandiol), 1 mM EDTA, 100 mM NaCl, 5 mM MgCl₂ und 1 Gew.-% Nonidet P 40 (ethoxylierter Fettalkohol, Firma Shell).

Die ATP-Bestimmungen erfolgten mit Hilfe des ATP Bioluminescence Assay Kit CLS II (Boehringer Mannheim). Das Testprinzip dieses Assays beruht darauf, dass die Luciferase von Photinus pyralis eine Reaktion katalysiert, bei der in Gegenwart von ATP D-Luciferin in Oxyluciferin umgewandelt wird. Bei dieser Reaktion wird grünes Licht emittiert, das mit einem Luminometer gemessen werden kann. Das emittierte Biolumineszenzlicht ist proportional zur Menge des vorhandenen ATP.

Zur Bestimmung des ATP-Gehalts wurden 50 µl des Zell-Lysats in eine schwarze Luminometer-Mikrotiterplatte pipettiert, mit 50 µl Luciferase-Reagens aus dem Assay Kit CLS II versetzt und sofort in das Luminometer eingesetzt, das die auftretende Biolumineszenz bei Raumtemperatur erfasst.

Die Eichung des Luminometers erfolgte durch Biolumineszenz-Messungen mit ATP-Standardlösungen im Konzentrationsbereich von 1,6 · 10⁻⁹ bis 1,0 · 10⁻⁶ mol ATP / I. Die Gleichung der Eichgeraden wurde zur Berechnung der ATP-Konzentration in den Zell-Lysaten herangezogen.

### 2.4 Überprüfung der ATP-Messungen mit Hilfe des MTT-Tests

Um zu überprüfen, inwieweit die ATP-Messungen durch mögliche zytotoxische Effekte der Testsubstanzen beeinträchtigt wurden, wurde die Vitalität der Zellkulturen durch einen Farbstofftest, den MTT-Test, überprüft.
Der MTT-Test liefert Informationen über die Zellproliferation und Zytotoxizität. Im Test wird die metabolische Aktivität lebender Zellen bestimmt. Die exakte Duchführung des Tests ist in J. Immunol. Methods 65, 55, 1983 (T. Mosmann) offenbart, worauf hier explizit Bezug genommen wird. Das Tetrazoliumsalz 3-[4,5-Dimethylthiazol-2-yl]-2,5-diphenyltetrazoliumbromid (MIT) wird in lebenden Zellen enzymatisch reduziert und in ein blaues, wasserunlösliches Formazansalz umgewandelt. Dieses Formazansalz wird extrahiert und photometrisch quantifiziert. Die Farbintensität der Formazansalz-Lösung kann mit der Anzahl lebender Zellen beziehungsweise mit der Vitalität eines Gewebes in der untersuchten Probe korreliert werden.

**Tabelle 5:**

| Wirkung der Spirulina-Extrakte auf die Vitalität (Zellzahl/Metalbolismus) von primären Keratinozyten: Angegeben ist die Veränderung der Vitalität unter Einfluss der Substanzen im Vergleich zur unbehandelten Kontrolle = 0% | | |
|---|---|---|
| **Substanz** | **Gehalt** | **24 Stunden** |
| | | **Vitalität:** **Abweichung von der Kontrolle in % (n=8)** |
| SPH 3000 | 0,1 Gew.-% | + 30 |
| | 0,5 Gew.-% | -103 |
| | 1,0 Gew.-% | -105 |
| SPHM 3001 | 0,5 Gew.-% | +18 |
| | 1,0 Gew.-% | +13 |
| SPHM 3002 | 0,1 Gew.-% | +46 |
| | 0,5 Gew.-% | + 42 |
| | 1,0 Gew.-% | + 27 |

Die einzelnen Algenextrakte beeinflussten die Vitalität der primären Keratinozyten nach 24stündiger Inkubation in unterschiedlichem Ausmaß. Mit zunehmender Konzentration der Prüfsubstanzen nahm die Vitalität der Zellen ab, gelangte jedoch nur bei Behandlung mit dem nicht erfindungsgemäßen Extrakt SPH 3000 in den Konzentrationen 0,5 Gew.-% und 1 Gew.-% in einen zytotoxischen Bereich. Der erfindungsgemäße Extrakt SPHM 3002 zeigte insbesondere in den Konzentrationen 0,1 Gew.-% und 0,5 Gew.-% einen positiven Effekt auf die Vitalität (Zellproliferation/Metabolismus) der Keratinozyten.

### 2.5 Ergebnisse der ATP-Messungen

Die Messungen des Einflusses der Spirulina-Extrakte SPH 3000, SPHM 3001 und SPHM 3002 auf den intrazellulären ATP-Gehalt der primären humanen Keratinozyten sind nachfolgend tabellarisch zusammengestellt. Angegeben sind die absoluten und prozentualen, auf die unbehandelte Kontrolle (= 100 %) bezogenen ATP-Gehalte nach Behandlung mit den Prüfsubstanzen. Bei den Werten handelt es sich um Mittelwerte aus 8 Einzelmessungen.

**Tabelle 6:**

| Einfluss der Spirulina-Extrakte SPH 3000, SPHM 3001 und SPHM 3002 auf den intrazellulären ATP-Gehaft primärer humaner Keratinozyten nach 3 und 6 Stunden Inkubation | | | | | | | |
|---|---|---|---|---|---|---|---|
| **Substanz** | **Konz. [Gew.-%]** | **3 Stunden** | | | **6 Stunden** | | |
| | | **ATP-Gehalt [µM]** | Standard-abweichung [SD] | **ATP-Gehalt [%]** | **ATP-Gehalt [µM]** | Standard-abweichung [SD] | **ATP-Gehalt [%]** |
| **unbehandelt (Vergleich)** | | **0,129** | 0,015 | 100 | **0,180** | 0,035 | 100 |
| **SPH 3000** | 0,1 | **0,144** | 0,015 | 111 | **0,148** | 0,017 | 83 |
| | 0,5 | **0,039** | 0,015 | 32 | **0,021** | 0,012 | 13 |
| | 1,0 | **0,005** | 0,001 | 4 | **0,001** | 0,000 | 1 |
| **SPHM 3001** | 0,5 | **0,203** | 0,032 | 154 | **0,243** | 0,041 | 133 |
| | 1,0 | **0,211** | 0,019 | 159 | **0,206** | 0,024 | 114 |
| **SPHM 3002** | 0,1 | **0,158** | 0,021 | 122 | **0,197** | 0,035 | 109 |
| | 0,5 | **0,154** | 0,016 | 119 | **0,172** | 0,020 | 96 |
| | 1,0 | **0,193** | 0,031 | 147 | **0,208** | 0,019 | 115 |

Der größte Effekt auf die ATP-Synthese wurde nach 3stündiger Inkubation beobachtet. Hier fand sich bei Behandlung mit SPHM 3001 gegenüber der unbehandelten Vergleichsprobe eine Stimulierung der ATP-Synthese um 54 % bei einem Gehalt an Algenextrakt von 0,5 Gew.-% bzw. um 59 % bei bei einem Gehalt an Algenextrakt von 1 Gew.-%. SPHM 3002 bewirkte eine ATP-Steigerung in ähnlichem Umfang um 47 % bei 1 Gew.-% und in geringerem Umfang in den Konzentrationen 0,1 Gew.-% und 0,5 Gew.-%.

Nach 6stündiger Inkubation war ebenfalls noch tendenziell für beide Extrakte ein Effekt auf die ATP-Synthese zu sehen. Dieser war am stärksten ausgeprägt bei Behandlung mit SPHM 3001 (33% Steigerung ATP) und mit SPHM 3002 1 Gew.-% (15 % Steigerung ATP).

Die Behandlung mit SPH 3000 bewirkte keine Steigerung des ATP-Gehalts. Bei der geringsten Konzentration von 0,1 Gew.-% lag der ATP-Gehalt im Bereich der Kontrolle, nahm aber mit steigender Konzentration weiter ab.

Die erfindungsgemäßen Algenextrakte SPHM 3001 und SPHM 3002 bewirkten nach 3-und 6stündiger Inkubationsdauer in primären Keratinozyten eine Steigerung des intrazellulären ATP-Gehaltes im Vergleich zur unbehandelten Kontrolle. Außerdem wirkte sich insbesondere SPHM 3002 positiv auf die Zellproliferation beziehungsweise den Metabolismus der Zellen aus.

## Patentansprüche

1. Kosmetische Verwendung eines wässrigen oder wässrig-alkoholischen Extraktes aus Blaualgen, der einen Gehalt an Magnesium von wenigstens 10 Gew.-% Mg, bezogen auf die Trockenmasse des Extrakts, aufweist, als Komponente zur Stimulierung der intrazellulären Synthese von Matrixproteinen und der Differenzierung von Keratinozyten in topischen Zubereitungen zur Reinigung und kosmetischen Pflege der Haut und der Haare.

2. Verwendung gemäß Anspruch 1 zur pflegenden Behandlung der trockenen Haut.

3. Verwendung gemäß Anspruch 1 zur pflegenden Behandlung der Altershaut.

4. Verwendung gemäß einem der vorhergehenden Ansprüche, **dadurch gekennzeichnet, dass** der Extrakt aus dem Blaualgentaxon Spirulina Arthrospira gewonnen wurde.

5. Verwendung gemäß einem der vorhergehenden Ansprüche, **dadurch gekennzeichnet, dass** der Extrakt eine Trockenmasse von 2 - 10 Gew.-% und einen Magnesiumgehalt von 0,3 - 2 Gew.-%, bezogen auf das Gewicht des Extrakts, aufweist.

## Claims

1. Cosmetic use of an aqueous or aqueous-alcoholic extract of blue algae which has a magnesium content of at least 10% by weight Mg, based on the dry weight of the extract, as a component for stimulating the intracellular synthesis of matrix proteins and differentiating keratinocytes in topical preparations for the cleaning and cosmetic care of the skin and hair.

2. Use claimed in claim 1 for the caring treatment of dry skin.

3. Use claimed in claim 1 for the caring treatment of ageing skin.

4. Use claimed in any of the preceding claims, **characterized in that** the extract is obtained from the blue alga taxon Spirulina Arthrospira.

5. Use claimed in any of the preceding claims, **characterized in that** the extract has a dry weight of 2 to 10% by weight and a magnesium content of 0.3 to 2% by weight, based on the weight of the extract.

## Revendications

1. Utilisation cosmétique d'un extrait aqueux ou aqueux-alcoolique d'algues bleues, qui présente une teneur en magnésium d'au moins 10 % en poids de Mg, rapportés à la matière sèche de l'extrait, à titre de composant pour la stimulation de la synthèse intracellulaire de protéines matricielles et de la différenciation de kératinocytes dans des formulations topiques pour le nettoyage et les soins cosmétiques de la peau et des cheveux.

2. Utilisation selon la revendication 1 pour le traitement de soin de la peau sèche.

3. Utilisation selon la revendication 1 pour le traitement de soin de la peau mature.

4. Utilisation selon l'une quelconque des revendications précédentes, **caractérisé en ce que** l'extrait a été obtenu à partir du taxon d'algue bleue Spirulina arthrospira.

5. Utilisation selon l'une quelconque des revendications précédentes, **caractérisée en ce que** l'extrait présente un pourcentage en matière sèche de 2 à 10 % en poids et une teneur en magnésium à concurrence de 0,3 à 2 % en poids, rapportés au poids de l'extrait.
